(19) Europäisches Patentamt
European Patent Office
Office européen des brevets

(11) Veröffentlichungsnummer: **0 090 388 B1**

(12) # EUROPÄISCHE PATENTSCHRIFT

(45) Veröffentlichungstag der Patentschrift: **08.05.85**

(51) Int. Cl.⁴: **C 07 K 5/00**, C 12 Q 1/36

(21) Anmeldenummer: **83103025.9**

(22) Anmeldetag: **26.03.83**

(54) **Chromophore Peptide, Verfahren zu ihrer Herstellung, diese enthaltende Mittel und ihre Verwendung zum Nachweis von DD-Carboxypeptidasen.**

(30) Priorität: **31.03.82 DE 3211932**

(43) Veröffentlichungstag der Anmeldung: **05.10.83 Patentblatt 83/40**

(45) Bekanntmachung des Hinweises auf die Patenterteilung: **08.05.85 Patentblatt 85/19**

(84) Benannte Vertragsstaaten: **AT BE CH DE FR GB IT LI NL SE**

(56) Entgegenhaltungen:
**COLLECTION OF CHECHOSLOVAK CHEMICAL COMMUNICATIONS, Band 32, 1967, Seiten 843-847, Ausg. Akademie VED M. ZAORAL et al.: "Amino acids and peptides. LXVIII. Preparation of three lysine-vasopressin analogs modified at position 9: D-ala9-lysine-vasopressin, ala9-lysine-vasopressin, and 9-ethylenediamine-lysine-vasopressin"**
**RECUEIL DES TRAVAUX CHIMIQUES DES PAYS-BAS, Band 83, Nr. 1, Januar 1964, Seiten 53, 54, 65, Ausg. W.P. van Stockum & Zoon, Den Haag, NL. G.I. TESSER et al.: "Synthesis of a completely protected pentapeptide found in bacterial cell walls"**

(73) Patentinhaber: **HOECHST AKTIENGESELLSCHAFT, Postfach 80 03 20, D-6230 Frankfurt am Main 80 (DE)**

(72) Erfinder: **Schindler, Peter, Dr., Reinhardswaldweg 1, D-6082 Mörfelden-Walldorf (DE)**
Erfinder: **König, Wolfgang, Dr., Eppsteiner Strasse 25, D-6238 Hofheim am Taunus (DE)**

EP 0 090 388 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft Verbindungen der allgemeinen Formel I

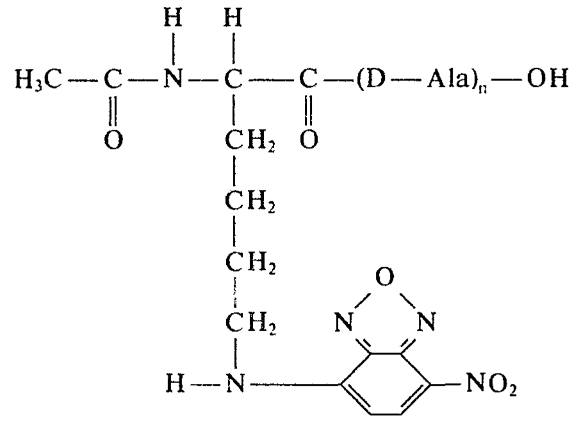

(I)

in der n für 1 oder 2 steht.

Der quantitativen Bestimmung von $\beta$-Lactam-Antibiotika in biologischen Systemen wie z. B. Blut, Urin, Nahrungsmitteln oder Fermentationsbrühen kommt erhebliche Bedeutung zu. Eine universell anwendbare Methode mit der Nanogramme $\beta$-Lactam pro ml zuverlässig bestimmt werden können, wurde kürzlich von Frère, J.-M. et al. (Antimicrob. Agents Chemother. 1980, 18, 506–510) beschrieben. Diese Methode nutzt die Eigenschaft von $\beta$-Lactam-Antibiotika aus, das Enzym DD-Carboxypeptidase aus Streptomyces R 39 unter Bildung eines stöchiometrischen Komplexes zu inaktivieren. Nach Titration der DD-Carboxypeptidase mit $\beta$-Lactamen wird die verbliebene Enzymaktivität bestimmt. Hierzu verwenden die Autoren $N^{\alpha}$, $N^{\varepsilon}$-Diacetyl-L-lysyl-D-alanyl-D-alanin, dessen terminales D-Alanin von der DD-Carboxypeptidase abgespalten wird. Das freigesetzte D-Alanin wird in einem D-Aminosäure-Oxidase/Peroxidase/o-Dianisidin-gekoppelten Testansatz quantitativ bestimmt.

Gegenüber einem solchen mehrfach gekoppelten Testansatz bieten chromophore Substrate wesentliche Vorteile, weil sich das Substrat und das den Chromophor tragende Produkt der Reaktion nach Trennung in einem geeigneten System, vorzugsweise mit Hilfe der Chromatographie, ohne weitere Vorkehrungen durch ihre charakteristische Eigenfarbe zu erkennen geben. Darüber hinaus entfallen grundsätzlich störende Einflüsse, wie sie für die Bestimmung von Reaktionsprodukten mittels gekoppelter Testansätze bekannt sind.

Überraschenderweise wurde nun festgestellt, daß das chromophorhaltige Tripeptid der Formel I, in der n für 2 steht, von der DD-Carboxypeptidase aus Streptomyces R 39 mit mindestens derselben Effizienz spezifisch gespalten wird wie $N^{\alpha}$, $N^{\varepsilon}$-Diacetyl-L-lysyl-D-alanyl-D-alanin. Damit kann die Empfindlichkeit der von Frère et al. angegebenen $\beta$-Lactam-Bestimmung mit Hilfe der erfindungsgemäßen Verbindung voll ausgenutzt werden.

Das neue chromophorhaltige Tripeptid ist intensiv gelb gefärbt und zeichnet sich darüber hinaus durch seine starke Fluoreszenz im langwelligen UV aus.

DD-Carboxypeptidase-Präparationen aus Streptomyces R 39 spalten von diesem Tripeptid (im folgenden auch als »Substrat« bezeichnet) in spezifischer Weise den C-terminalen D-Alaninrest unter Bildung des den Chromophor tragenden erfindungsgemäßen Dipeptids der Formel I mit n = 1 (im folgenden auch als »Produkt« bezeichnet) ab, das sich von der erfindungsgemäßen Verbindung der Formel I (n = 2) leicht beispielsweise durch Chromatographie an geeignetem Trägermaterial abtrennen läßt.

Die Erfindung betrifft weiterhin ein Verfahren zur Herstellung von chromophoren Peptiden der allgemeinen Formel I, das dadurch gekennzeichnet ist, daß man $N^{\alpha}$-Acetyl, N-[4-(7-nitrobenzofurazanyl)]-L-Lysin (= Ac-Lys (NBF)-OH) mit einer Verbindung der allgemeinen Formel II

$$H-(D-Ala)_n-X, \qquad (II)$$

in der X OH oder $OBu^t$ bedeutet und n für 1 oder 2 steht umsetzt und anschließend im Falle des tert.-Butylesters die tert.-Butylgruppe vorzugsweise sauer abspaltet.

Die Kondensation von Ac-Lys (NBF)-OH mit Peptiden ist besonders kritisch, da $\alpha$-Acetyl-aminosäuren bei der Aktivierung ihrer Carboxylgruppe wegen der möglichen Azlactonbildung zur Racemisierung neigen. Es dürfen also für die Kondensation nur Methoden verwendet werden, die nur wenig Racemisierung zeigen. Bei der Umsetzung von Ac-Lys (NBF)-OH mit H-D-Ala-D-Ala-OH muß Ac-Lys (NBF)-OH zunächst voraktiviert werden, da die freie Carboxylgruppe von H-D-Ala-D-Ala-OH störend wirken würde. Für die racemisierungsfreie Voraktivierung von Peptiden bzw. Aminosäurederivaten, die wegen ihrer Tendenz zur Azlactonbildung zur Racemisierung neigen, empfiehlt sich neben der Azid-Methode vor allem die Voraktivierung mit Dicyclohexylcarbodiimid (DCC) und 3-Hydroxy-4-oxo-3,4-dihydro-1,2,3-benzotriazin (HOObt) (Chem. Ber. 103, 2034–2040, 1970). Hierbei wird in unserem Fall Ac-Lys

(NBF)-OH mit molaren Mengen HOObt vorzugsweise in dipolar aprotischen Lösungsmitteln wie beispielsweise Tetrahydrofuran, Dimethylformamid, Dimethylacetamid oder ähnlichen gelöst und bei −10°C bis +10°C, vorzugsweise bei −2° bis +2°C mit DCC versetzt.

Man läßt etwa 1 bis 2 Stunden reagieren und gibt dann die Aminokomponente H-D-Ala-D-Ala-OH zu, rührt bis alles gelöst ist und arbeitet dann in geeigneter Weise auf.

Bei der Umsetzung von Ac-Lys (NBF)-OH mit H-D-Ala-D-Ala-$OBu^t$ kann man auch einfacher vorgehen und sogenannte »Eintopfmethoden« benutzen. Hierbei wird die Carboxyl- und die Aminokomponente in einem geeigneten Lösungsmittel gelöst und das Kondensationsmittel zugegeben. Auch hier ist wieder mit Racemisierung zu rechnen, so daß auch hier nur Methoden mit geringer Neigung zur Racemisierung in Frage kommen. Hier empfiehlt sich wegen der einfachen Durchführung die DCC-Methode unter Zusatz von verschiedenen möglichen N-Hydroxyverbindungen wie z. B. 1-Hydroxybenzotriazol (HOBt), HOObt oder N-Hydroxysuccinimid. Die zur Durchführung des erfindungsgemäßen Verfahrens erforderlichen Ausgangssubstanzen sind literaturbekannt oder können nach literaturbekannten Verfahren hergestellt werden.

Die Erfindung betrifft ferner ein Verfahren zum Nachweis von DD-Carboxypeptidasen, das dadurch gekennzeichnet ist, daß man eine Lösung der Verbindung der Formel I, in der n = 2 ist, mit der die DD-Carboxypeptidasen enthaltenden Analysenprobe inkubiert, die entstandene Verbindung der Formel I, in der n = 1 ist, abtrennt und deren Menge quantitativ bestimmt.

Die Erfindung betrifft auch die Verwendung der chromophoren Peptide der allgemeinen Formel I zum Nachweis von DD-Carboxypeptidase-Inhibitoren und zum Nachweis von $\beta$-Lactamen nach Vorinkubation mit DD-Carboxypeptidasen, sowie Peptide der Formel I enthaltende Mittel.

Zweckmäßigerweise verfährt man bei der quantitativen Bestimmung der DD-Carboxypeptidasen so, daß man geeignet gepufferte wäßrige Lösungen des Tripeptids der Formel I mit DD-Carboxypeptidasen inkubiert. Beispielsweise hat sich ein 50-mM-Tris-HCl-Puffer von pH 8,3, der 0,1 M NaCl und 5 mM $MgCl_2$ enthält, als geeignet erwiesen. Nach Ablauf einer bestimmten Zeit, die im wesentlichen von der Menge des zu bestimmenden Enzyms abhängt, wird ein aliquoter Teil auf eine Kieselgelplatte aufgetragen.

Als Referenzsubstanz für das Reaktionsprodukt wird das Dipeptid der Formel I (n = 1) aufgetragen. Nach der chromatographischen Trennung in einem geeigneten System wie beispielsweise n-Butanol/Eisessig/n-Heptan, vorzugsweise im Volumenverhältnis 10 : 8 : 7, kann die Menge des gebildeten Reaktionsprodukts quantitativ bestimmt werden. Die Auswertung kann visuell oder streng quantitativ durch dem Fachmann wohlbekannte densitometrische Methoden erfolgen.

In dieser Weise kann das erfindungsgemäße Tripeptid auch zum Nachweis der DD-Carboxypeptidase in Kulturbrühen von Streptomyceten, wie z. B. Streptomyces R 39, bei der Reinigung dieses Enzyms sowie zum Nachweis von DD-Carboxypeptidase-Inhibitoren herangezogen werden. Einer DD-Carboxypeptidase-Inhibitoren enthaltenden Probe wird ein bekannter Überschuß an DD-Carboxypeptidase zugesetzt. Die Inhibitormenge läßt sich dann aus der Differenz der zugesetzten DD-Carboxypeptidase und deren Restaktivität ermitteln.

Der Nachweis der DD-Carboxypeptidase-Aktivität bei der Bestimmung von $\beta$-Lactamen erfolgt auch direkt, d. h. ohne einen gekoppelten Testansatz, da sich auch hier das erfindungsgemäße Substrat und das hieraus entstehende Dipeptid nach der chromatographischen Trennung durch die für den Chromophor typische Eigenfarbe bzw. Fluoreszenz zu erkennen geben.

Ein besonderer Vorteil der vorliegenden Erfindung ist jedoch, daß für die Bestimmung der nach Vorbehandlung mit $\beta$-Lactamhaltigen Lösungen verbliebenen DD-Carboxypeptidase-Aktivität das erfindungsgemäße Substrat und das durch die Einwirkung von DD-Carboxypeptidase ggf. entstandene chromophorhaltige Dipeptid nicht mit Hilfe aufwendiger Apparaturen wie z. B. HPLC getrennt werden müssen. Die Trennung wird vielmehr schon auf einfachen Dünnschichtplatten wie z. B. Kieselgel F 254 (Merck) und einem geeigneten Laufmittel, vorzugsweise n-Butanol-Eisessig-n-Heptan (10 : 8 : 7), erreicht. Für die semiquantitative Beurteilung der verbliebenen DD-Carboxypeptidase-Aktivität genügt eine visuelle Beurteilung der Intensität des durch das erfindungsgemäße Substrat bzw. Reaktionsprodukt bedingten intensiv gelben Flecks, bzw. durch die im langwelligen ultravioletten Licht sichtbare grüne Fluoreszenz dieser Verbindungen auf dem Chromatogramm.

Von Vorteil ist weiterhin, daß die Verwendung des erfindungsgemäßen Substrats die Bestimmung eines der beiden Reaktionsprodukte (D-Alanin) mittels eines mehrfach gekoppelten Testansatzes überflüssig macht. Dies ist für einen wichtigen Anwendungsbereich dieses Testes, die $\beta$-Lactam-Bestimmung in Kulturbrühen von Mikroorganismen, besonders wertvoll, da solche Systeme erfahrungsgemäß häufig mit dem gekoppelten Testansatz in der Weise interferieren, daß eine Bestimmung des enzymatisch gebildeten D-Alanins unmöglich wird. Daher ist die Verwendung des erfindungsgemäßen Tripeptids für den Nachweis von $\beta$-Lactamen in komplexen biologischen Systemen, insbesondere in Fermentationsbrühen von Mikroorganismen besonders wertvoll.

Ein weiterer Vorteil der erfindungsgemäßen Nachweisverfahren kann darin gesehen werden, daß auf einfache Weise eine große Zahl von Testen nebeneinander ausgeführt und auf einer einzigen, entsprechend dimensionierten Dünnschichtplatte gleichzeitig ausgewertet werden kann.

## Beispiel 1

### Ac-Lys(NBF)-D-Ala-D-Ala-OH

#### a) Ac-Lys (NBF)-OH

Zu einer Lösung von 0,77 g N-Acetyl-L-Lysin (4,1 mmol) in 200 ml 0,6 N $NaHCO_3$ gibt man bei Raumtemperatur eine Suspension von 1,08 g 7-Chlor-4-nitrobenzofurazan (5,4 mmol) in 250 ml Methanol. Man rührt bei Raumtemperatur über Nacht. Anderntags wird Methanol am Rotationsverdampfer bei 40°C entfernt und die wäßrige Phase mehrfach mit Essigester extrahiert. Die wäßrige Phase wird mit 6 N Salzsäure auf pH 2 angesäuert, mit Essigester mehrfach extrahiert, die so erhaltene Essigester-Phase wird über Natriumsulfat getrocknet und eingeengt. Durch Chromatographie an Kieselgel in Essigester/Isopropanol/Wasser (4 : 3 : 2) wird die Substanz rein isoliert.

Ausbeute 960 mg. Schmp. 119–122°C.

$[\alpha]_D^{23} = -56,6°$ (c = 1, 50% Essigsäure).

#### b) H-D-Ala-D-Ala-$OBu^t$ · HCl

Zu einer Lösung von 4,46 g (20 mmol) Z-D-Ala-OH, 3,87 g H-D-Ala-$OBu^t$ · HCl und 2,7 g HOBt in 50 ml Dimethylformamid gibt man bei 0°C 2,6 ml N-Äthylmorpholin und 4,4 g DCC. Man rührt zwei Stunden bei 0°C und läßt über Nacht bei Raumtemperatur stehen. Anderntags wird der Niederschlag abgesaugt und das Filtrat im Hochvakuum eingeengt. Der Rückstand wird zwischen Wasser und Essigester verteilt. Die Essigesterphase wird mit einem $KHSO_4/K_2SO_4$-Puffer und anschließend mit gesättigter $NaHCO_3$-Lösung und Wasser ausgeschüttelt, über Natriumsulfat getrocknet und eingeengt.

Ausbeute: 8,7 g.

Zur Reinigung wird die Substanz in Methylenchlorid/Aceton (0,5 : 0,5) über 800 g Kieselgel chromatographiert.

Ausbeute 6,7 g einer öligen Substanz.

Die oben gewonnenen 6,7 g Z-D-Ala-D-Ala-$OBu^t$ werden in 300 ml Methanol gelöst und nach Zugabe von Pd/Kohle-Katalysator am Autotitrator unter Zugabe von ca. 1 N methanolischer HCl katalytisch hydriert. Nach beendigter Hydrierung wird der Katalysator abgesaugt und das Filtrat eingeengt und im Hochvakuum getrocknet. Es bleiben 4,1 g einer amorphen, hygroskopischen, aber DC-einheitlichen Substanz zurück.

$[\alpha]_D^{24} = +36,0°$ (c = 0,5, Methanol)

DC: $R_f = 0,65$ in n-Butanol/Eisessig/Wasser (8 : 2 : 2)

#### c) Ac-Lys (NBF)-D-Ala-D-Ala-OH

Zu einer Lösung von 1,4 g Ac-Lys (NBF)-OH, 1,08 g H-D-Ala-D-Ala-$OBu^t$ · HCl und 540 mg HOBt in 10 ml absolutem Tetrahydrofuran gibt man bei 0°C 0,5 ml N-Ethylmorpholin und 880 mg DDC. Man läßt 2 Std. bei 0°C rühren und über Nacht bei Raumtemperatur stehen. Anderntags wird mit 100 ml Essigester der Ansatz verdünnt und der Niederschlag abgesaugt. Das Filtrat wird mit Wasser, gesättigter $NaHCO_3$-Lösung, $KHSO_4/K_2SO_4$-Puffer und nochmals mit gesättigter $NaHCO_3$-Lösung ausgeschüttelt, über Natriumsulfat getrocknet und eingeengt. Der Rückstand wird mit etwa 20 ml Methylenchlorid/Methanol (19 : 1) verrührt. Dabei kristallisiert eine Substanz aus, die abgesaugt wird.

Ausbeute 810 mg, Schmp. 120–125°C.

Nach DC in Methylenchlorid/Methanol (19 : 1) ist die Substanz einheitlich. In der Mutterlauge ist noch Ac-Lys(NBF)-D-Ala-D-Ala-$OBu^t$ vorhanden. Durch Chromatographie an Kieselgel in Methylenchlorid/Methanol (19 : 1) wird die Substanz rein isoliert.

Ausbeute 150 mg. Gesamtausbeute an Ac-Lys(NBF)-D-Ala-D-Ala-$OBu^t$ : 960 mg.

Die oben gewonnenen 960 mg Ac-Lys(NBF)-D-Ala-D-Ala-$OBu^t$ werden in 20 ml 90%iger Trifluoressigsäure gelöst. Man läßt eine Stunde bei Raumtemperatur stehen, engt ein und löst den Rückstand in Wasser. Von noch Unlöslichem wird abgesaugt und das Filtrat gefriergetrocknet.

Ausbeute 530 mg, Schmp. 121°C (sublimiert teilweise),

$[\alpha]_D^{27} = +20,8°$ (c = 1, in 50%iger Essigsäure).

Laut Elementaranalyse liegt das Peptid als Monohydrat vor.

$C_{20}H_{27}N_7O_8 \cdot H_2O$ (511,5)

| | | | |
|---|---|---|---|
| Berechnet: | C 46,95 | H 5,71 | N 19,17 |
| Gefunden: | C 46,3 | H 5,7 | N 18,6 |

## Beispiel 2

### Ac-Lys(NBF)-D-Ala-D-Ala-OH · $CH_3COOH$

#### a) H-D-Ala-D-Ala-OH

5,4 g H-D-Ala-D-Ala-$OBu^t$ · HCl werden in 25 ml halbkonzentrierter Salzsäure gelöst und sofort im Hochvakuum eingeengt. Der Rückstand wird in wenig Methanol gelöst und mit N-Ethylmorpholin die saure Lösung sofort neutralisiert. Durch Zugabe von Methylenchlorid kann das Dipeptid ausgefällt werden. Man läßt vier Stunden bei 0°C stehen, saugt ab und wäscht mit Methylenchlorid nach.

Ausbeute 2,15 g, Schmp. 246—248°C
$[\alpha]_D^{28}$ + 17,6° (c = 1, Wasser).

#### b) Ac-Lys(NBF)-D-Ala-D-Ala-OH · $CH_3COOH$

Zu einer Lösung von 351 mg Ac-Lys (NBF)-OH und 163 mg HOOBt in 3 ml absolutem Tetrahydrofuran gibt man bei 0°C 210 mg DCC, läßt zwei Stunden bei 0°C und eine Stunde bei Raumtemperatur rühren und gibt dann 160 mg H-D-Ala-D-Ala-OH und 3 ml Dimethylformamid zu. Man läßt noch 4 Stunden bei Raumtemperatur rühren und läßt über Nacht bei Raumtemperatur stehen. Anderntags wird eingeengt und der Rückstand mit heißem Wasser verrieben. Von Unlöslichem wird abgesaugt und das Filtrat wird eingeengt. Der Rückstand wird an Kieselgel in n-Butanol/Eisessig/n-Heptan (10 : 5 : 5) chromatographiert. Die Fraktionen, die Ac-Lys(NBF)-D-Ala-D-Ala-OH rein enthalten, werden vereinigt und eingeengt. Der Rückstand wird in Wasser gelöst und gefriergetrocknet.

Ausbeute 180 mg, Schmp. 164°C

Nach DC in n-Butanol/Eisessig/n-Heptan (10 : 5 : 5) einheitlich und identisch mit dem nach Beispiel 1 hergestelltem Derivat. Im NMR-Spektrum sieht man zwei Acetylgruppen, die dafür sprechen, daß das Peptid als Acetat vorliegt.

Ein weiterer Hinweis, daß die Substanz als Acetat vorliegt ist die Tatsache, daß das nach Beispiel 1 hergestellte Peptid, nachdem es in Essigsäure gelöst war und wieder gefriergetrocknet wurde, einen Schmelzpunkt von 165°C hatte.

## Beispiel 3

### Ac-Lys(NBF)-D-Ala-OH (als Chromatographievergleich)

Zu einer Lösung von 702 mg Ac-Lys(NBF)-OH, 400 mg H-D-Ala-$OBu^t$ · HCl und 270 mg HOBt in 6 ml Dimethylformamid gibt man bei 0°C 0,26 ml Dimethylformamid und 440 mg DCC. Man läßt zwei Stunden bei 0°C rühren und über Nacht bei Raumtemperatur stehen. Anderntags wird der Ansatz mit 50 ml Essigester verdünnt und mit Wasser, gesättigter $NaHCO_3$-Lösung, $KHSO_4/K_2SO_4$-Puffer und gesättigter $NaHCO_3$-Lösung ausgeschüttelt. Die Essigesterphase wird über $Na_2SO_4$ getrocknet und eingeengt. Der Rückstand wird an Kieselgel in Methylenchlorid/Methanol (20 : 1) chromatographiert.

Ausbeute 400 mg.

Die gewonnenen 400 mg Ac-Lys(NBF)-D-Ala-$OBu^t$ werden in 10 ml 90%iger Trifluoressigsäure gelöst. Man läßt eine Stunde bei Raumtemperatur stehen und engt ein. Der Rückstand wird in Wasser gelöst und gefriergetrocknet.

Ausbeute 300 mg, Schmp. 112°C, $[\alpha]_D^{32}$ = +0,9° (c = 1, 50%ige Essigsäure).

Laut Elementaranalyse liegt das Peptid als Monohydrat vor.

$C_{17}H_{22}N_6O_7 \cdot H_2O$ (440,4)

Berechnet: C 46,3 H 5,49 N 19,08
Gefunden: C 45,6 H 5,2 N 18,6

**Patentansprüche für die Vertragsstaaten: BE, CH, DE, FR, GB, IT, LI, NL, SE**

1. Chromophore Peptide der allgemeinen Formel I

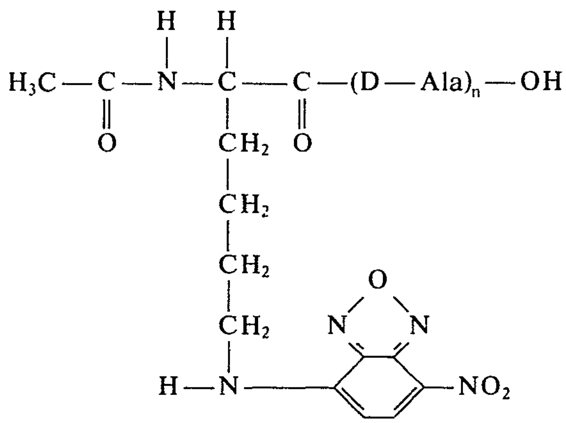

(I)

in der n für 1 oder 2 steht.

2. Verfahren zur Herstellung von chromophoren Peptiden der allgemeinen Formel I gemäß Anspruch 1, dadurch gekennzeichnet, daß man $N^{\alpha}$-Acetyl, N-[4-(2-nitrobenzofurazanyl)]-L-Lysin mit einer Verbindung der allgemeinen Formel II

$$H-(D-Ala)_n-X\,, \qquad (II)$$

in der X OH oder $OBu^t$ bedeutet und n für 1 oder 2 steht, umsetzt und anschließend im Falle des tert.-Butylesters die tert.-Butylgruppe abspaltet.

3. Verfahren zum Nachweis von DD-Carboxypeptidasen, dadurch gekennzeichnet, daß man eine Lösung der Verbindung der Formel I, in der n = 2 ist, mit der die DD-Carboxypeptidase enthaltenden Analysenprobe inkubiert, die entstandene Verbindung der Formel I, in der n = 1 ist, abtrennt und deren Menge quantitativ bestimmt.

4. Verfahren gemäß Anspruch 3, dadurch gekennzeichnet, daß man die Analysenprobe, die DD-Carboxypeptidase-Inhibitoren enthält, eine überschüssige Menge an DD-Carboxypeptidase zusetzt und die DD-Carboxypeptidase-Inhibitor-Menge aus der Hemmung der DD-Carboxypeptidase-Aktivität berechnet.

5. Verfahren gemäß Anspruch 3, dadurch gekennzeichnet, daß man die β-Lactam enthaltenden Analysenprobe mit DD-Carboxypeptidasen vorinkubiert und den β-Lactamgehalt aus der DD-Carboxypeptidase-Restaktivität ermittelt.

6. Verwendung von chromophoren Peptiden der allgemeinen Formel I gemäß Anspruch 1 zum Nachweis von DD-Carboxypeptidasen.

7. Verwendung von chromophoren Peptiden der allgemeinen Formel I gemäß Anspruch 1 zum Nachweis von DD-Carboxypeptidase-Inhibitoren.

8. Verwendung von chromophoren Peptiden der allgemeinen Formel I gemäß Anspruch 1 zum Nachweis von β-Lactamen nach Vorinkubation mit DD-Carboxypeptidasen.

9. Mittel enthaltend chromophore Peptide der allgemeinen Formel I gemäß Anspruch 1.

**Patentansprüche für den Vertragsstaat: AT**

1. Verfahren zur Herstellung von chromophoren Peptiden der allgemeinen Formel I

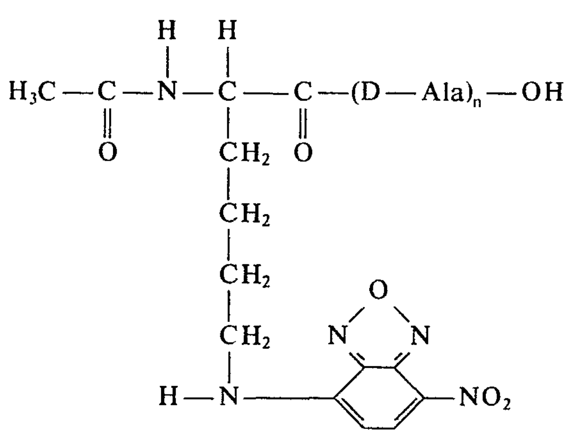

(I)

in der n für 1 oder 2 steht, dadurch gekennzeichnet, daß man $N^{\alpha}$-Acetyl, N-[4-(2-nitrobenzofurazanyl)]-L-Lysin mit einer Verbindung der allgemeinen Formel II

$$H-(D-Ala)_n-X, \qquad (II)$$

in der X OH oder $OBu^t$ bedeutet und n für 1 oder 2 steht, umsetzt und anschließend im Falle des tert.-Butylesters die tert.-Butylgruppe abspaltet.

2. Verfahren zum Nachweis von DD-Carboxypeptidasen, dadurch gekennzeichnet, daß man eine Lösung der Verbindung der Formel I, in der n = 2, ist, mit der die DD-Carboxypeptidase enthaltenden Analysenprobe inkubiert, die entstandene Verbindung der Formel I, in der n = 1 ist, abtrennt und deren Menge quantitativ bestimmt.

3. Verfahren gemäß Anspruch 2, dadurch gekennzeichnet, daß man die Analysenprobe, die DD-Carboxypeptidase-Inhibitoren enthält, eine überschüssige Menge an DD-Carboxypeptidase zusetzt und die DD-Carboxypeptidase-Inhibitor-Menge aus der Hemmung der DD-Carboxypeptidase-Aktivität berechnet.

4. Verfahren gemäß Anspruch 2, dadurch gekennzeichnet, daß man die $\beta$-Lactam enthaltenden Analysenprobe mit DD-Carboxypeptidasen vorinkubiert und den $\beta$-Lactamgehalt aus der DD-Carboxypeptidase-Restaktivität ermittelt.

5. Verwendung von chromophoren Peptiden der allgemeinen Formel I gemäß Anspruch 1 zum Nachweis von DD-Carboxypeptidasen.

6. Verwendung von chromophoren Peptiden der allgemeinen Formel I gemäß Anspruch 1 zum Nachweis von DD-Carboxypeptidase-Inhibitoren.

7. Verwendung von chromophoren Peptiden der allgemeinen Formel I gemäß Anspruch 1 zum Nachweis von $\beta$-Lactamen nach Vorinkubation mit DD-Carboxypeptidasen.

8. Verfahren zur Herstellung eines Mittels, enthaltend chromophore Peptide der allgemeinen Formel I, dadurch gekennzeichnet, daß man die Peptide der allgemeinen Formel I in eine geeignete Zubereitungsform bringt.

**Claims for the contracting states: BE, CH, DE, FR, GB, IT, LI, NL, SE**

1. A chromophoric peptide of the general formula I

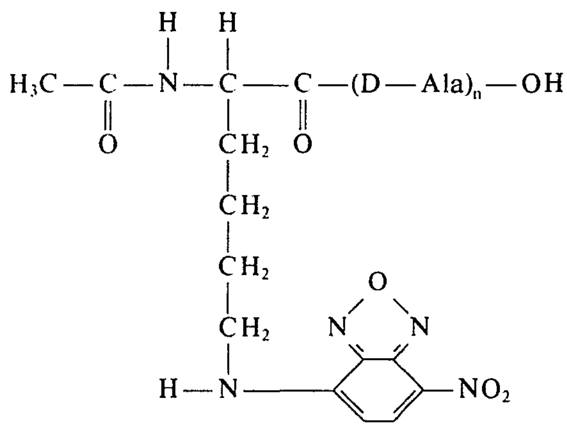

(I)

in which n represents 1 or 2.

2. A process for the preparation of chromophoric peptides of the general formula I as claimed in claim 1, which comprises reacting N $\alpha$-acetyl-N-[4-(2-nitrobenzofurazanyl)]-L-lysine with a compound of the general formula II

$$H-(D-Ala)_n-X, \qquad (II)$$

in which X denotes OH or $OBu^t$ and n represents 1 or 2, and then, in the case of the tert.-butyl ester, cleaving off the tert.-butyl group.

3. A procedure for determining DD-carboxypeptidases, which comprises incubating a solution of the compound of the formula I in which n is 2 with the sample for analysis containing the DD-carboxypeptidase, separating off the resulting compound of the formula I in which n is 1, and quantitatively determining the amount of it.

4. The procedure as claimed in claim 3, wherein an excess amount of DD-carboxypeptidase is added to the sample for analysis, which contains DD-carboxypeptidase inhibitors, and the amount of DD-carboxypeptidase inhibitors is calculated from the inhibition of the DD-carboxypeptidase activity.

5. The procedure as claimed in claim 3, wherein the sample for analysis containing $\beta$-lactam is

pre-incubated with DD-carboxypeptidases and the $\beta$-lactam content is found from the residual DD-carboxypeptidase activity.

6. The use of chromophoric peptides of the general formula I as claimed in claim 1 for determining DD-carboxypeptidases.

7. The use of chromophoric peptides of the general formula I as claimed in claim 1 for determining DD-carboxypeptidase inhibitors.

8. The use of chromophoric peptides of the general formula I as claimed in claim 1 for determining $\beta$-lactams after pre-incubation with DD-carboxypeptidases.

9. An agent containing chromophoric peptides of the general formula I as claimed in claim 1.

**Claims for the contracting state: AT**

1. A process for the preparation of chromophoric peptides of the general formula I

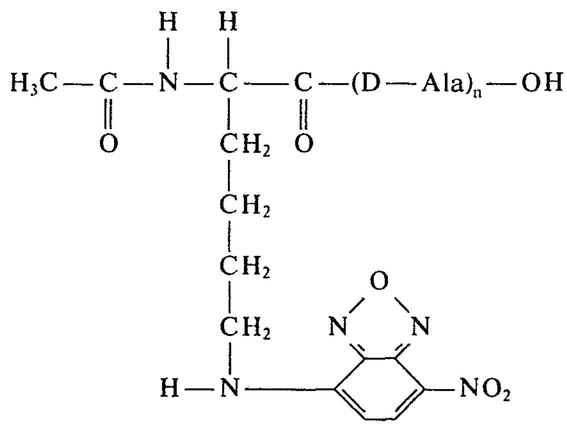

(I)

in which n represents 1 or 2, which comprises reacting N$\alpha$-acetyl-N-[4-(2-nitrobenzofurazanyl)]-L-lysine with a compound of the general formula II

$$H-(D-Ala)_n-X, \qquad (II)$$

in which X denotes OH or $OBu^t$ and n represents 1 or 2, and then, in the case of the tert.-butyl ester, cleaving off the tert.-butyl group.

2. A procedure for determining DD-carboxypeptidases, which comprises incubating a solution of the compound of the formula I in which n is 2 with the sample for analysis containing the DD-carboxypeptidase, separating off the resulting compound of the formula I in which n is 1, and quantitatively determining the amount of it.

3. The procedure as claimed in claim 2, wherein an excess amount of DD-carboxypeptidase is added to the sample for analysis, which contains DD-carboxypeptidase inhibitors, and the amount of DD-carboxypeptidase inhibitors is calculated from the inhibition of the DD-carboxypeptidase activity.

4. The procedure as claimed in claim 2, wherein the sample for analysis containing $\beta$-lactam is pre-incubated with DD-carboxypeptidases and the $\beta$-lactam content is found from the residual DD-carboxypeptidase activity.

5. The use of chromophoric peptides of the general formula I as claimed in claim 1 for determining DD-carboxypeptidases.

6. The use of chromophoric peptides of the general formula I as claimed in claim 1 for determining DD-carboxypeptidase inhibitors.

7. The use of chromophoric peptides of the general formula I as claimed in claim 1 for determining $\beta$-lactams after pre-incubation with DD-carboxypeptidases.

8. A process for the preparation of an agent containing chromophoric peptides of the general formula I characterized in that a peptide of the general formula I is brought in a suitable form of composition.

**Revendications pour les Etats contractants: BE, CH, DE, FR, GB, IT, LI, NL, SE**

1. Peptides chromophores de formule générale I

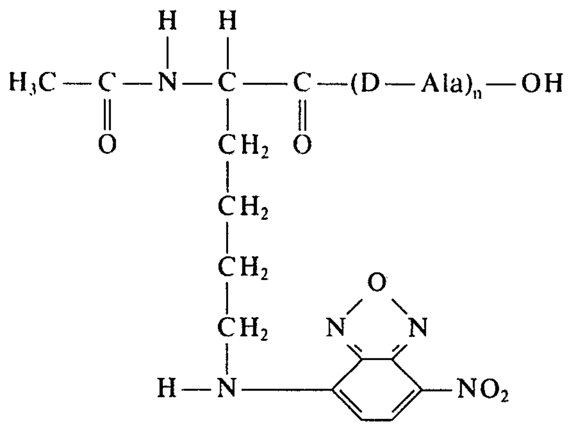

(I)

dans laquelle n est 1 ou 2.

2. Procédé pour la préparation de peptides chromophores de formule générale I selon la revendication 1, caractérisé en ce qu'on fait réagir la $N^{\alpha}$-acétyl, N-[4-(2-nitrobenzofurazanyl)]-L-lysine avec un composé de formule générale II

H–(D–Ala)$_n$–X, (II)

dans laquelle X représente OH ou $OBu^t$ et n est 1 ou 2, puis, dans le cas de l'ester tert-butylique, on élimine le groupe butyle tertiaire.

3. Procédé de détection de DD-carboxypeptidases, caractérisé en ce qu'on incube une solution du composé de formule I, dans lequel n est 2, avec l'échantillon à analyser contenant la DD-carboxypeptidase, on sépare le composé de formule I formé, dans lequel n est 1 et on détermine quantitaviment sa quantité.

4. Procédé selon la revendication 3, caractérisé en ce que, à l'échantillon à analyser qui contient des inhibiteurs de DD-carboxypeptidase, on ajoute une quantité en excès de DD-carboxypeptidase et on calcule la quantité d'inhibiteur de DD-carboxypeptidase à partir de l'inhibition de l'activité de la DD-carboxypeptidase.

5. Procédé selon la revendication 3, caractérisé en ce qu'on pré-incube l'échantillon à analyser contenant des $\beta$-lactames avec des DD-carboxypeptidases et on détermine la teneur en $\beta$-lactames à partir de l'activité résiduelle de la DD-carboxypeptidase.

6. Utilisation de peptides chromophores de formule générale I selon la revendication 1 pour la détection de DD-carboxypeptidases.

7. Utilisation de peptides chromophores de formule générale I selon la revendication 1 pour la détection d'inhibiteurs de DD-carboxypeptidases.

8. Utilisation de peptides chromophores de formule générale 1 selon la revendication 1 pour la détection de $\beta$-lactames après une pré-incubation avec des DD-carboxypeptidases.

9. Agent contenant des peptides chromophores de formule générale I selon la revendication 1.

**Revendications pour l'Etat contractant: AT**

1. Procédé pour la préparation de peptides chromophores de formule générale I

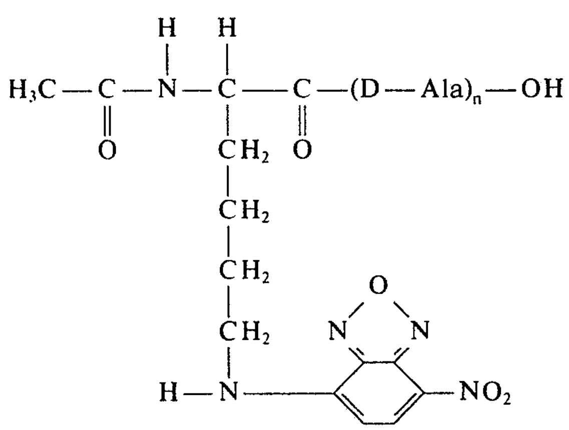

(I)

dans laquelle n est 1 ou 2, caractérisé en ce qu'on fait réagir la $N^{\alpha}$-acétyl, N-[4-(2-nitrobenzofurazanyl)]-L-lysine avec un composé de formule générale II

$$H-(D-Ala)_n-X\,, \qquad (II)$$

dans laquelle X est OH ou $OBu^t$ et n est 1 ou 2, puis, dans le cas de l'ester tert-butylique, on élimine le groupe butyle tertiaire.

2. Procédé de détection de DD-carboxypeptidases, caractérisé en ce qu'on incube une solution de composé de formule I, dans lequel n est 2, avec l'échantillon à analyser contenant la DD-carboxypeptidase, on sépare le groupe de formule I formé, dans lequel n est 1, et on détermine quantitativement sa quantité.

3. Procédé selon la revendication 2, caractérisé en ce qu'à l'echantillon à analyser qui contient des inhibiteurs de DD-carboxypeptidases, on ajoute une quantité en excès de DD-carboxypeptidase et on calcule la quantité d'inhibiteurs de DD-carboxypeptidase à partir de l'inhibition de l'activité de la DD-carboxypeptidase.

4. Procédé selon la revendication 2, caractérisé en ce qu'on pré-incube l'échantillon à analyser contenant des $\beta$-lactames, avec des DD-carboxypeptidases et on détermine la teneur en $\beta$-lactames à partir de l'activité résiduelle de la DD-carboxypeptidase.

5. Utilisation de peptides chromophores de formule générale I selon la revendication 1 pour la détection de DD-carboxypeptidases.

6. Utilisation de peptides chromophores de formule générale I selon la revendication 1 pour la détection d'inhibiteurs de DD-carboxypeptidases.

7. Utilisation de peptides chromophores de formule générale I selon la revendication 1 pour la détection de $\beta$-lactames après une pré-incubation avec des DD-carboxypeptidases.

8. Procédé pour la préparation d'un agent, contenant des peptides chromophores de formule générale I, caractérisé en ce qu'on met les peptides de formule générale I sous une forme de préparation appropriée.